# EUROPEAN PATENT SPECIFICATION

(11) **EP 3 313 365 B1**
(45) Date of publication and mention of the grant of the patent: **18.12.2024**
(21) Application number: 15731581.3
(22) Date of filing: 24.06.2015
(51) Int. Cl.: A61K 8/81, A61Q 5/04, A61Q 5/06, A61Q 5/00

(54) **HAIR CONDITIONING COMPOSITION**
HAARKONDITIONIER-ZUSAMMENSETZUNG
COMPOSITION POUR CONDITIONNER DES CHEVEUX

(43) Date of publication of application: 02.05.2018
(73) Proprietor: Kao Germany GmbH, 64297 Darmstadt (DE)
(72) Inventor: HOFFMANN, Martin, 64297 Darmstadt Hessen (DE); NING, Jing, 64297 Darmstadt Hessen (DE)
(86) International application number: PCT/EP2015/064287
(87) International publication number: WO 2016/206739

(56) References cited:
- EP-A1- 1 787 634
- EP-A1- 2 011 478
- EP-A1- 2 191 864
- WO-A1-2009/102758
- WO-A2-2013/087316
- US-A1- 2007 154 441
- T V DROVETSKAYA ET AL: "New high-charge density hydrophobically modified cationic HEC polymers for improved co-deposition of benefit agents and serious conditioning for problem hair", 31 August 2007 (2007-08-31), pages 421 - 434, XP055235466, Retrieved from the Internet <URL:http://journal.scconline.org//pdf/cc2007/cc058n04/p00421-p00434.pdf> [retrieved on 20151210]

## Description

The present invention relates to an aqueous composition for treating hair comprising one or more cationic polymers having certain cationic charge density and one or more of nonionic surfactants. The novel composition provides hair long lasting conditioning, especially improves combability and suppleness of hair which is held up to 20 hair washes.

Hair conditioners are widely used for improving hair combability and suppleness among other properties which are as well improved and/or given. Consumers use the conditioners after each shampooing as hair combability and suppleness is usually reduced after cleansing the hair surface. Such process takes long time under shower and therefore there is a great need for hair conditioners which provides improved and hair long lasting combability and suppleness. In other words, the consumers do not need to use a conditioning composition after each hair shampooing for improving combability and suppleness for certain number of hair washes.

The above-mentioned problems have been subject of numerous research projects. The one approach has been improving hair conditioning properties of cleansing compositions so that there is no need for using an additional composition just for making hair better combable and supple. In this way there have been many product initiatives on the market in form of so-called two-in-one shampoo compositions.

DE 10 2013225898 discloses compositions for long lasting hair conditioning comprising cationic polymer with a relatively high charge density. It has been observed that compositions disclosed therein do not really provide the long-lasting conditioning effects because of several reasons which are made clear below.

The inventors of the present invention have surprisingly found out that an aqueous composition comprising cationic quaternary ammonium polymer having certain cationic charge density, one or more nonionic surfactants and one or more aminated silicones provides hair long lasting conditioning effect, especially in terms of combability and suppleness, which is held with repeated hair washes up to 20 wash cycles.

Accordingly, the first object of the present invention is an aqueous composition as described in claim 1..

Second object of the present invention is the use of the aqueous composition for long lasting conditioning of human hair, especially improving combability and suppleness of hair.

The third object of the present invention is a kit for treating human hair comprising the aqueous composition of the present invention and a device which makes the increase of hair temperature possible.

The fourth object is the process for long lasting conditioning of human hair wherein the aqueous composition of the present invention is applied onto hair, optionally left on the hair for a period of 1 to 15 min, optionally rinsed off from hair and the hair temperature is increased to the range of 40 to 140°C.

The aqueous composition of the present invention comprises one or more cationic quaternary ammonium polymers having a charge density of 3 mEq/g or more, preferably 3.5 to 8 mEq/g, more preferably 4 to 7.5 mEq/g, most preferably 4.5 to 7.0 mEq and in particular 4.5 to 6.5 mEq/g. In a preferred embodiment of the present invention the aqueous composition comprises only one cationic quaternary ammonium polymer with the above defined cationic charge density ranges.

Suitable non-limiting cationic quaternary ammonium polymers for the purpose of the present invention are Polyquaternium-37, Polyquaternium-6, Polyquaternium-7 and Polyquaternium-16.

The preferred cationic polymers are Polyquaternium-37 and Polyquaternium-16. The particularly preferred cationic polymer is Polyquaternium-37

Total cationic quaternary ammonium polymer concentration having the above-described cationic charge density is in the range of 0.1 to 5%, preferably 0.2 to 4%, more preferably 0.25 to 3% and most preferably 0.3 to 2.5% by weight, calculated to the total composition.

The aqueous composition of the present invention comprises one or more nonionic surfactants. Suitable ones are fatty alcohol ethoxylates of the following general structure

R₁ (OCH₂CH₂)ₙ OH

wherein R₁ is straight or branched, saturated or unsaturated alkyl chain which may be synthetic or natural with a C chain length in the range of 8 to 40, preferably 9 to 30 and more preferably 9 to 24 and n is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

R₂ (OCH₂ (CH₃) CH₂)ₙ OH

wherein R₂ is straight or branched, saturated or unsaturated fatty alcohol which may be synthetic or natural with a C chain length in the range of 8 to 40, preferably 9 to 30 and more preferably 9 to 24 and n is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R₃ C(O) (OCH₂CH₂)ₙ OH

wherein R₃ is straight or branched, saturated or unsaturated alkyl group which may be synthetic or natural with a C chain length in the range of 7 to 39, preferably 9 to 29 and more preferably 9 to 23 and n is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R₄ C(O) (OCH₂(CH₃) CH₂)ₙ OH

wherein R₄ is straight or branched, saturated or unsaturated alkyl group which may be synthetic or natural with a C chain length in the range of 7 to 39, preferably 9 to 29 and more preferably 9 to 23 and n is a number in the range of 1 to 40, preferably 9 to 30.

Suitable non-limiting examples are PPG-15 Isostearate, PPG-9 Laurate, PPG-26 Oleate and PPG-36 Oleate.

Further nonionic suitable surfactants are polyethylene glycol and polypropylene glycol ether of fatty alcohols of the following general structure

R₅ (OCH2 (CH3) CH₂)ₙ₁ (OCH2CH2)n2 OH

wherein R₅ is straight or branched, saturated or unsaturated alkyl group which may be synthetic or natural with a C chain length in the range of 7 to 39, preferably 9 to 29 and more preferably 9 to 23 and n1 and n2 may be the same or different and are a number in the range of 1 to 40.

Suitable non-limiting examples are PPG-2 Ceteareth-9, PPG-4 Ceteareth-12, PPG-4 Ceteareth-20, PPG-2 C9 - 11 Pareth-5, PPG-2 C9 - 11 Pareth-7, PPG-2 C9 - 11 Pareth-8, PPG-2 C9 - 11 Pareth-11, PPG-2 C12 - 13 Pareth-8, PPG-2 C12 - 15 Pareth-6, PPG-4 C 13- 15 Pareth-15, PPG-5 C9 - 15 Pareth-6, PPG-6 C9 - 11 Pareth-5, PPG-6 C12 - 15 Pareth-12, PPG-6 C12 - 18 Pareth-11, PPG-1 Deceth-4, PPG-1 Deceth-5, PPG-1 Deceth-6, PPG-1 Deceth-7, PPG-2 Deceth-3, PPG-2 Deceth-7, PPG-2 Deceth-8, PPG-2 Deceth-10, PPG-2 Deceth-15, PPG-2 Deceth-20, PPG-2 Deceth-30, PPG-2 Deceth-40, PPG-4 Deceth-4, PPG-4 Deceth-6, PPG-4 Deceth-6, PPG-6 Deceth-4, PPG-6 Deceth-9, PPG-8 Deceth-6, PPG-14 Deceth-6, PPG-2 Laureth-5, PPG-2 Laureth-8, PPG-2 Laureth-12, PPG-3 Laureth-8, PPG-3 Laureth-9, PPG-3 Laureth-10, PPG-3 Laureth-12, PPG-4 Laureth-2, PPG-4 Laureth-5, PPG-4 Laureth-7, PPG-4 Laurreth-15, PPG-5 Laureth-5, PPG-5 Laureth-3, PPG-20 Laureth-12, PPG-25 Laureth-25, PPG-3 Myreth-3, PPG-3 Myreth-11, PPG-9 Steareth-3, PPG-23 Steareth-34, PPG-30 Steareth-4, PPG-34 Steareth-3, and PPG-38 Steareth-6.

Total concentration of non-ionic surfactants is in the range of 0.1 to 5%, preferably 0.2 to 4%, more preferably 0.25 to 3% and most preferably 0.3 to 2.5% by weight, calculated to the total composition.

The aqueous composition of the present invention comprises one or more aminated silicones according to the general structure 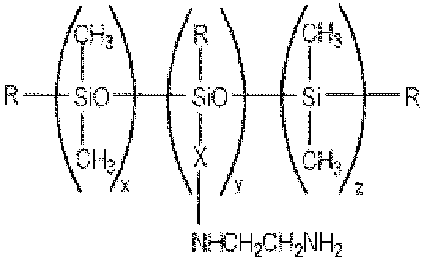 wherein R is the same or different OH or CH₃ or OCH₃ and X represents butyl, propyl, isopropyl or isobutyl.

The especially preferred aminated silicone is Bis(Hydroxyl/Methoxy) Amodimethicone wherein X is isobutyl and one of the R is OH and the other is OCH₃ in the above general structure.

Total concentration of one or more aminated silicones is in the range of 0.1 to 5%, preferably 0.2 to 4%, more preferably 0.25 to 3% and most preferably 0.3 to 2.5% by weight, calculated to the total composition.

In a preferred embodiment of the present invention the aqueous composition of the present invention does not comprise quaternary ammonium surfactants at high concentrations. It has been observed that when quaternary ammonium surfactants are comprised in the composition at a concentration above 10% preferably 5% of the total concentration of the one or more cationic polymers as defined above, the long-lasting effect of the aqueous composition is dramatically diminished. Without being bound by the theory, one explanation might be that the amount of adsorbed / bound cationic polymer concentration is decreased by co-adsorption of the cationic surfactants. Accordingly, the aqueous composition of the present invention does not comprise quaternary ammonium surfactants at a concentration of 10%, preferably 5% or more, by weight, of the total concentration of cationic polymer as defined above.

Specifically, the aqueous composition of the present invention does not comprise quaternary ammonium surfactant of the general structure below at a concentration more than 10%, preferably 5% by weight of the total concentration of one or more cationic quaternary ammonium polymers having the cationic charge density as defined above. The general structure of cationic quaternary ammonium surfactant is
R₁₁ R₁₂ R₁₃ R₁₄ N
wherein R₁₁ is an alky chain having C length of 8 to 30 which may be saturated or unsaturated, straight or branched, R₁₂ is an alky chain having C length of 1 to 30 which may be saturated or unsaturated, straight or branched, R₁₁ and R₁₂ additionally may take the structures of

R₁₅ C(O) O (CH2) n or R₁₅ C(O) NH (CH2)n

wherein R₁₅ is an alkyl chain with a C length of 7 to 29 which may be saturated or unsaturated, straight or branched and n is a number between 1 and 4,

R₁₃ and R₁₄ are same or the different alkyl chain with a C length of 1 to 4 which may be straight or branched (only for C₃ and C₄), wherein all alkyl chains may comprise one or more substituents such as hydroxyl or (poly)ethoxy groups.

The composition of the present invention may comprise one or more fatty alcohols and may be in a form of emulsion. Suitable non-limiting examples are according to general structure

R₆-OH

wherein R₆ is straight or branched, saturated or unsaturated alkyl chain which may be synthetic or natural with a C chain length in the range of 12 to 24 which may as well be substituted with one or more groups on the alkyl chain.

Non limiting suitable examples are lauryl alcohol, myristyl alcohol, cetyl alcohol, oleyl alcohol, stearyl alcohol, behenyl alcohol and their mixtures. As a mixed fatty alcohol the mostly used one is the cetearyl alcohol as well the preferred fatty alcohol in the compositions of the present invention.

The total concentration of one or more fatty alcohols is in the range of 0.1 to 20%, preferably 0.5 to 15%, more preferably 1 to 10% and the most preferably 2 to 7.5% by weight calculated to the total composition.

The aqueous composition of the present invention may comprise one or more organic solvents. Suitable ones are ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are ethanol, isopropanol, benzylalcohol and polypropylene glycols. Concentration of organic solvents should not exceed 20%, preferably in the range of 0.1 to 15 %, more preferably 0.1 to 10% by weight and most preferably 0.1 to 7.5% by weight calculated to total composition. It should be noted that concentration of one or more organic solvents is very much dependent on the type of preparation i.e. a solution can contain higher concentration of organic solvent than a gel or emulsion composition.

Further in an embodiment of the present invention, the aqueous composition of the present invention comprises at least one hair direct dye selected from cationic and neutral nitro dyes.

Any cationic direct dye is in principal suitable for the compositions. Non-limiting suitable examples are Basic Blue 6, Basic Blue 7, Basic Blue 9, Basic Blue 26, Basic Blue 41, Basic Blue 99, Basic Blue 124, Basic Brown 4, Basic Brown 7, Basic Brown 16, Basic Brown 17, Basic Green 1, Basic Orange 31, Basic Red 2, Basic Red 12, Basic Red 22, Basic Red 51, Basic Red 76, Basic Violet 2, Basic Violet 3, Basic Violet 10, Basic Violet 14, Basic Violet 57 and Basic Yellow 87.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Total concentration of one or more direct dyes is in the range of 0.001 to 1.0% by weight, preferably 0.01 to 0.8% more preferably 0.05 to 0.75% and most preferably 0.1 to 0.5% by weight calculated to total composition.

The aqueous composition of the present invention may further comprise one or more UV filters which may be selected from water soluble ones as well as oils soluble ones. The oil soluble UV filter are more preferred ones as they show no interaction with the cationic quaternary ammonium polymers. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15.

The total UV filter concentration may be in the range of 0.01 to 1 % by weight calculated to the total composition.

The composition of the present invention may comprise oil and/or oily substances which are suitably selected from silicone oils, either volatile or non-volatile, natural and synthetic oils, fatty alcohol ethers (dialkyl ethers) and fatty acid fatty alcohol esters. Among silicone oils those can be added to the compositions include, dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, aqueous emulsion of divinyldimethicone/dimethicone copolymer, preferably with a viscosity of higher than 1 × 10⁸ mm²/s, more preferably higher than 1.1 × 10⁸ mm²/s measured at 0.01 Hz and at approximately 25°C.

Suitable non-limiting examples to natural plant oils are such as olive oil, almond oil, avocado oil, wheatgerm oil and ricinus oil.

Suitable non-limiting examples to fatty alcohol fatty acid esters are such as isopropyl myristate, palmitate, stearate and isostearate, oleyl oleate, isocetyl stearate, hexyl laurate, dibutyl adipate, dioctyl adipate, myristyl myristate and oleyl erucate.

Suitable non-limiting examples to fatty alchol ethers (dialkyl ethers) are such as dimyristyl ether, dicetyl ether and dicaprylyl ether.

The compositions comprise oil and/or oily substances at a total concentration in the range of 0.01 to 10%, preferably 0.05 to 7.5%, more preferably 0.1 to 5 and most preferably 0.1 ta 3% by weight calculated to total composition.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

The composition of the present invention may further comprise natural plant extracts. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known **per se** are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon^{®}" products, "Herbasol^{R} ", "Sedaplant^{R}" and "Hexaplant^{R}". Extracts and the preparation thereof are also described in "Hagers Handbuch der pharmazeutischen Praxis", 4^{th} Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The natural plant extracts are included into the compositions at a concentration of 0.001 to 1.0%, preferably 0.005 to 0.75%, more preferably 0.01 to 0.6% and most preferably 0.05 to 0.5% by weight, calculated to total composition based on dry matter of the extract.

Further in an embodiment of the present invention, compositions comprise one or more ubiquinone of the following general structure 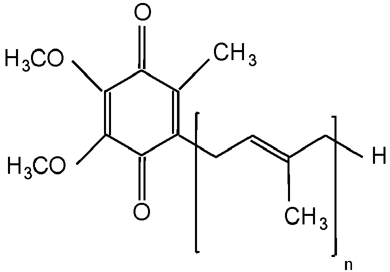 where n is a number between 1 and 10. It should be noted that the compositions of the present invention can certainly comprise more than one ubiquinone. Preferred ubiquinones are the ones where n is a number between 6 and 10 and especially preferred is Ubiquinone 50 where n is 10, also known as Coenzyme Q10.

Concentration ubiquinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The pH of the compositions according to the present invention is suitably between 3.0 and 8.0 and preferably in the range of 3.5 to 6.5, more preferably 4.0 to 5.5 and most preferably 4.0 to 5.

In principal pH of the compositions can be adjusted with any organic and/or inorganic acids or their mixture. Some of them to mention are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Conditioning compositions of the present invention can comprise additionally any compound customarily found in conditioning compositions such as chelating agents, preservatives and fragrance.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Viscosity of the conditioning composition may be adjusted according to the application form and generally should not be more than 50,000 mPa.s at 20°C measured with Brookfield Rheometer at a shear rate of 5 sec⁻¹.

Thickening agents especially the nonionic thickening polymers may be comprised in the compositions of the present invention. Suitable non-limiting examples are cellulose derivatives such as hydroxyethyl cellulose, methyl cellulose, hydroxymethyl cellulose, hydroxypropyl cellulose, guar gum and its derivatives, and cognac mannan and derivatives. Thickeners may be included at a concentration of 0.05 to 2.5% by weight calculated to total composition. Concentration of thickener is very much dependent on the thickener itself and also the preparation such as pH value of the composition etc. and therefore should be selected depending on the desired viscosity of the composition.

The aqueous composition of the present invention may be in the form of solution, gel, dispersion and emulsion. These preparations may be provided to the consumers as they are confectioned in a suitable packaging which allows easy release of the products and distribution onto hair as well as they may be provided in a pressurized container. In case pressurized container is used as the vessel carrying the composition, it additionally comprises a pressurizing gas which may be selected form the any known ones such as alkanes , butane, isobutene, dimethyl ether, pressurized air, nitrogen and carbon dioxide.

The compositions of the present invention is used in a process for long lasting conditioning hair wherein the hair temperature is increased to the range of 40 to 140°C, preferably 60 to 140°C, more preferably 70 to 130°C, most preferably 80 to 120°C and in particular 90 to 110°C. The heating of the hair is achieved with a hair dryer, with digital hair heating devices, with an iron which may be either flat or having certain surface structures or a round shaped iron. The operation of such kind of devices is commonly known by the skilled in the art or by the users who may be seen as average users of such devices.

In a preferred embodiment of the process the aqueous composition is not rinsed off from the hair prior to increasing the temperature of the hair.

The following examples are to illustrate the invention but not to limit it.

### Example 1

The following compositions were prepared for the comparative tests.

| | % active matter | | |
|---|---|---|---|
| | A | B | C |
| Polyquaternium 37 | 0.8 | 0.8 | 0.8 |
| C12-13 Pareth-9 | - | 0.5 | 0.5 |
| PPG-3 Caprylyl ether | - | - | 1.0 |
| Bis (Hydroxy/Methoxy) Amodimethicone | - | - | 0.5 |
| Citric acid Water | q.s. to pH 4.0 to 100 | | |

The composition C is within the scope of the claim 1.

Human hair streaks having approximately 25 cm length and weighing approximately 3.5 g were used for the comparative tests. The streaks were damaged prior to the tests by means of double bleaching with a bleaching composition commercially available under the brand name Goldwell.

The streaks, always in duplicate, were treated with the above compositions. Therefore, each streak was applied approximately 1 g of the product and left on the streak for 15 min and the streaks were rinsed off with water. Afterwards only one of the streaks of each pair was dried with a hair drier. Hair temperature reached was approximately 95°C. The temperature measurement was carried out using an infrared measuring device from a distance of approximately 5 cm.

Additionally two hair streaks were treated with composition C and the one was heated using hair drier to a temperature of 95°C without rinsing off the composition (C+* in the Table below) and the other was treated with an hot iron operated at 130°C for 5 times which resulted in approximate hair temperature of 100°C (C+** in the Table below).

The suppleness of the hair was measured with a zwick machine and expressed as the force (in milliNewton - mN) to pull a streak through the rods of the machine. The machine is commercially available from Zwick/Roell Company and includes a manual. It was used according to its manual. Briefly, the streak was places among 5 metal rods which were placed offset in a given distance horizontally (2 cm) and vertically (8 cm) and the force needed to pull the streaks through the metal rods at a given rate (5 cm/min) was measured. All values reported in the Table below are average of at least 6 readings.

In order to test the long lastingness of hair suppleness, the hair streaks were washed 5 times first and the suppleness was measured again as described above and after 5 additional washes the measurements were repeated. For washing hair streaks, 5% by weight Sodium lauryl ether sulfate solution in water at pH 5.0 was used.

The following results were obtained.

| | | Suppleness (mN) | | |
|---|---|---|---|---|
| Composition | Heat | Start | 5 Wash | 10 Wash |
| Water | - | 1741 | 2072 | 1938 |
| A | + | 756 | 873 | 1023 |
| A | - | 791 | 1145 | 1578 |
| B | + | 697 | 809 | 798 |
| B | - | 715 | 1046 | 1311 |
| C | + | 450 | 515 | 659 |
| C | - | 525 | 1020 | 1280 |
| C | +* | 348 | 518 | 629 |
| C | +** | 388 | 489 | 572 |

It should be noted that the lower the value, the higher the suppleness of the hair.

From the above results it is clear that the aqueous composition according to the present invention improves hair suppleness and this effect is long lasting especially when hair temperature is increased after application of the composition. It is also beyond any doubt that addition of nonionic surfactant to the Polyqauternium-37 comprising composition improved additionally the suppleness of the hair. It was observed that the suppleness was affected by the presence of additional substances but the streaks treated with the composition of the present invention were judged to have enough suppleness.

The above results prove beyond any doubt the improved suppleness of the hair treated with the aqueous composition of the present invention.

The following examples fall within the scope of the present invention.

### Example 2

| | % by weight |
|---|---|
| Polyquaternium-37 | 0.80 |
| Ceteareth 30 | 1.25 |
| Cetearyl alcohol | 2.00 |
| Behenyl alcohol | 2.80 |
| Basic Red 51 | 0.20 |
| Basic Orange 37 | 0.02 |
| HC red no 3 | 0.08 |
| Basic yellow 87 | 0.03 |
| HC Yellow 2 | 0.03 |
| Bis hydroxyl methoxy amodimethicone | 0.85 |
| Almond oil | 0.10 |
| Vitis vinifera extract | 0.10 (dry matter) |
| Lactic acid | q.s. to pH 4.5 |
| Water | to 100 |

## Claims

1. An aqueous composition for human hair **characterized in that** comprises one or more cationic quaternary ammonium polymers having a cationic charge density of 3.0 mEq/g or more and one or more nonionic surfactants selected from
a- fatty alcohol ethoxylates of the following general structure
R₁ (OCH₂CH₂)ₙ OH
wherein R₁ is straight or branched, saturated or unsaturated alkyl chain which may be synthetic or natural with a C chain length in the range of 8 to 40,
b- polypropylene glycol ether of fatty alcohols according to general structure
R₂ (OCH₂ (CH₃) CH₂)ₙ OH
wherein R₂ is straight or branched, saturated or unsaturated fatty alcohol which may be synthetic or natural with a C chain length in the range of 8 to 40,
c- polyethylene glycol fatty acid esters of the following general structure
R₃ C(O) (OCH₂CH₂)ₙ OH
wherein R₃ is straight or branched, saturated or unsaturated alkyl group which may be synthetic or natural with a C chain length in the range of 7 to 39,
d- polypropylene glycol fatty acid esters of the following general structure
R₄ C(O) (OCH₂(CH₃) CH₂)ₙ OH
wherein R₄ is straight or branched, saturated or unsaturated alkyl group which may be synthetic or natural with a C chain length in the range of 7 to 39,
e- polyethylene glycol and polypropylene glycol ether of fatty alcohols of the following general structure
R₅ (OCH₂ (CH₃) CH₂)ₙ₁ (OCH₂CH₂)ₙ₂ OH
wherein R₅ is straight or branched, saturated or unsaturated alkyl group which may be synthetic or natural with a C chain length in the range of 7 to 39, and n₁ and n₂ may be the same or different and are a number in the range of 1 to 40
at a total concentration in the range of 0.2 to 4% by weight, calculated to the total composition, and
one or more aminated silicones according to general structure 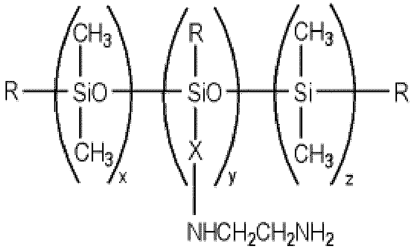
wherein R is the same or different OH or CH₃ or OCH₃ and X represents butyl, propyl, isopropyl or isobutyl, and
it has a pH in the range of 3.0 to 5.0.

2. The composition according to claim 1 **characterized in that** the cationic quaternary ammonium polymer has a charge density of 3.5 to 8 mEq/g,.

3. The composition according to claims 1 and 2 **characterized in that** the cationic quaternary ammonium polymer is selected from Polyquaternium-37, Polyquaternium-6, Polyquaternium-7, and Polyquaternium-16, preferably it is Polyquaternium-37.

4. The composition according to any one of the claims 1 to 3 **characterized in that** it comprises one or more cationic quaternary ammonium polymers at a total concentration of 0.1 to 5% by weight calculated to the total composition.

5. The composition according to any one of the claims 1 to 4 **characterized in that** one or more nonionic surfactants are selected from
fatty alcohol ethoxylates of the following general structure
R₁ (OCH₂CH₂)ₙ OH
wherein R₁ is straight or branched, saturated or unsaturated alkyl chain which may be synthetic or natural with a C chain length in the range of 8 to 40,.

6. The composition according to any one of the claims 1 to 5 **characterized in that** it comprises one or more nonionic surfactant at a total concentration of 0.25 to 3% and by weight, calculated to the total composition.

7. The composition according to one of the claims 1 to 6 **characterized in that** it comprises one or more aminated silicones according to general structure 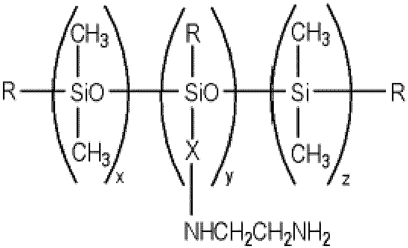 wherein R is the same or different OH or CH₃ or OCH₃ and X represents butyl, propyl, isopropyl or isobutyl at a concentration in the range of 0.1 to 5% by weight calculated to the total composition.

8. The composition according to one of the claims 1 to 7 **characterized in that** the aminated silicone is Bis(Hydroxy/Methoxy) Amodimethcone.

9. The composition according to one of the claims 1 to 8 **characterized in that** it does not comprise quaternary ammonium surfactant at a concentration more than 10%, by weight of the total concentration of one or more cationic quaternary ammonium polymers having the cationic charge density defined in the preceding claims.

10. The composition according to any of one of the proceeding claims **characterized in that** it comprises one or more of the following compounds
- Organic solvent,
- Fatty alcohol of the general structure
Re-OH
wherein R₆ is straight or branched, saturated or unsaturated alkyl chain which may be synthetic or natural with a C chain length in the range of 12 to 24 which may as well be substituted with one or more groups on the alkyl chain,
- UV filter,
- Hair direct dye selected from cationic and neutral dyes,
- Natural plant extract,
- Protein hydrolyzate,
- Ubiquinone, and
- Nonionic polymer.
- Oil and/or oily substances selected from silicone oils, natural and synthetic oily, fatty alcohol dialkyl ethers and fatty acid fatty alcohol esters.

11. The composition according to any one of the proceeding claims **characterized in that** it has a pH in the range of 4.0 to 5.0.

12. Use of the composition according to any of the preceding claims for long lasting conditioning of human hair, especially improving combability and suppleness of human hair.

13. A process for long lasting conditioning of human hair **characterized in that** an aqueous composition according to claims 1 to 11 is applied onto hair, optionally left on the hair for a period of 1 to 15 min, optionally rinsed off from hair and the hair temperature is increased to the range of 40 to 140°C,.

14. The process according to claim 13 **characterized in that** the hair is heated up with a hair dryer, with a digital hair heating device, with an iron which may be either flat or having certain surface structures or a round shaped iron.

15. Kit for hair **characterized in that** it comprises an aqueous composition according to the claims 1 to 11 and a device which makes the increase of hair temperature possible.

## Patentansprüche

1. Wässrige Zusammensetzung für menschliches Haar, **dadurch gekennzeichnet, dass** sie ein oder mehrere kationische quaternäre Ammoniumpolymere mit einer kationischen Ladungsdichte von 3.0 mEq/g oder mehr und ein oder mehrere nichtionische Tenside, ausgewählt aus
a- Fettalkoholethoxylaten mit der folgenden allgemeinen Struktur
R₁ (OCH₂CH₂)ₙ OH
worin R₁ eine gerade oder verzweigte, gesättigte oder ungesättigte Alkylkette ist, die synthetisch oder natürlich sein kann, mit einer C-Kettenlänge im Bereich von 8 bis 40,
b- Polypropylenglykolether von Fettalkoholen mit allgemeiner Struktur
R₂ (OCH₂ (CH₃)CH₂)ₙ OH
worin R₂ ein geradkettiger oder verzweigter, gesättigter oder ungesättigter Fettalkohol ist, der synthetisch oder natürlich sein kann und eine C-Kettenlänge im Bereich von 8 bis 40 aufweist,
c- Polyethylenglykol-Fettsäureester der folgenden allgemeinen Struktur
R₃ C(O) (OCH₂CH₂)ₙ OH
worin R₃ eine geradkettige oder verzweigte, gesättigte oder ungesättigte Alkylgruppe ist, die synthetisch oder natürlich sein kann und eine C-Kettenlänge im Bereich von 7 bis 39 aufweist,
d- Polypropylenglykolfettsäureester der folgenden allgemeinen Struktur
R₄ C(O) (OCH₂(CH₃)CH₂)ₙ OH
worin R₄ eine geradkettige oder verzweigte, gesättigte oder ungesättigte Alkylgruppe ist, die synthetisch oder natürlich sein kann und eine C-Kettenlänge im Bereich von 7 bis 39 aufweist,
e- Polyethylenglykol und Polypropylenglykolether von Fettalkoholen der folgenden allgemeinen Struktur
R₅ (OCH₂ (CH )s CH₂ )ₙ₁ (OCH₂CH₂)ₙ₂OH
worin R₅ eine geradkettige oder verzweigte, gesättigte oder ungesättigte Alkylgruppe ist, die synthetisch oder natürlich sein kann, mit einer C-Kettenlänge im Bereich von 7 bis 39, und n₁ und n₂ gleich oder verschieden sein können und eine Zahl im Bereich von 1 bis 40 sind in einer Gesamtkonzentration im Bereich von 0.2 bis 4 Gew.-%, berechnet auf die Gesamtzusammensetzung, und
ein oder mehrere aminierte Silikone mit der allgemeinen Struktur 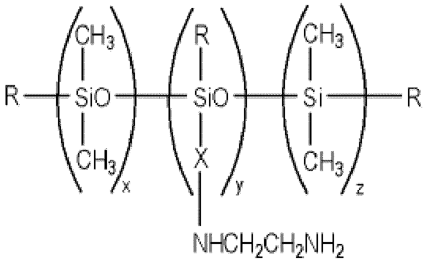
worin R gleich oder verschieden OH oder CH₃ oder OCH₃ ist und X Butyl, Propyl, Isopropyl oder Isobutyl darstellt, und
sie einen pH-Wert zwischen 3.0 und 5.0 hat.

2. Zusammensetzung nach Anspruch 1, **dadurch gekennzeichnet, dass** das kationische quaternäre Ammoniumpolymer eine Ladungsdichte von 3.5 bis 8 mEq/g aufweist.

3. Zusammensetzung nach den Ansprüchen 1 und 2, **dadurch gekennzeichnet, dass** das kationische quaternäre Ammoniumpolymer ausgewählt ist aus Polyquaternium-37, Polyquaternium-6, Polyquaternium-7 und Polyquaternium-16, vorzugsweise ist es Polyquaternium-37.

4. Zusammensetzung nach einem der Ansprüche 1 bis 3, **dadurch gekennzeichnet, dass** sie ein oder mehrere kationische quaternäre Ammoniumpolymere in einer Gesamtkonzentration von 0.1 bis 5 Gew.-%, berechnet auf die Gesamtzusammensetzung, enthält.

5. Zusammensetzung nach einem der Ansprüche 1 bis 4, **dadurch gekennzeichnet, dass** ein oder mehrere nichtionische Tenside ausgewählt sind aus
Fettalkoholethoxylate mit der folgenden allgemeinen Struktur
R₁ (OCH₂CH₂)ₙ OH
worin R₁ eine gerade oder verzweigte, gesättigte oder ungesättigte Alkylkette ist, die synthetisch oder natürlich sein kann, mit einer C-Kettenlänge im Bereich von 8 bis 40.

6. Zusammensetzung nach einem der Ansprüche 1 bis 5, **dadurch gekennzeichnet, dass** sie ein oder mehrere nichtionische Tenside in einer Gesamtkonzentration von 0.25 bis 3 Gew.-%, berechnet auf die Gesamtzusammensetzung, enthält.

7. Zusammensetzung nach einem der Ansprüche 1 bis 6, **dadurch gekennzeichnet, dass** sie ein oder mehrere aminierte Silikone mit der allgemeinen Struktur 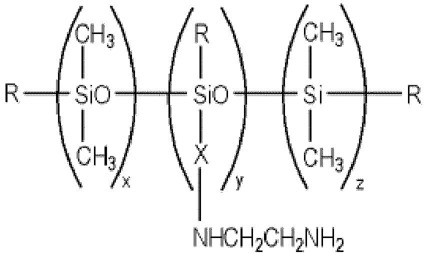 worin R gleich oder verschieden OH oder CH₃ oder OCH₃ ist und X Butyl, Propyl, Isopropyl oder Isobutyl darstellt, in einer Konzentration im Bereich von 0.1 bis 5 Gew.-%, berechnet auf die Gesamtzusammensetzung, enthält.

8. Zusammensetzung nach einem der Ansprüche 1 bis 7, **dadurch gekennzeichnet, dass** das aminierte Silikon Bis(Hydroxy/Methoxy)-amodimethicone ist.

9. Zusammensetzung nach einem der Ansprüche 1 bis 8, **dadurch gekennzeichnet, dass** sie kein quaternäres Ammoniumtensid in einer Konzentration von mehr als 10 Gew.-% der Gesamtkonzentration eines oder mehrerer kationischer quaternärer Ammoniumpolymere mit der in den vorhergehenden Ansprüchen definierten kationischen Ladungsdichte enthält.

10. Zusammensetzung nach einem der vorangehenden Ansprüche, **dadurch gekennzeichnet, dass** sie eine oder mehrere der folgenden Verbindungen enthält
- Organisches Lösungsmittel,
- Fettalkohol der allgemeinen Struktur
R-OH₆
worin R₆ eine gerade oder verzweigte, gesättigte oder ungesättigte Alkylkette ist, die synthetisch oder natürlich sein kann, mit einer C-Kettenlänge im Bereich von 12 bis 24, die auch mit einer oder mehreren Gruppen an der Alkylkette substituiert sein kann,
- UV-Filter,
- Haardirektfarbstoff, ausgewählt aus kationischen und neutralen Farbstoffen,
- Natürlicher Pflanzenextrakt,
- Proteinhydrolysat,
- Ubichinon, und
- Nichtionisches Polymer.
- Öle und/oder ölige Stoffe, ausgewählt aus Silikonölen, natürlichen und synthetischen Ölen, Fettalkoholdialkylethern und Fettsäurefettalkoholestern.

11. Zusammensetzung nach einem der vorangehenden Ansprüche, **dadurch gekennzeichnet, dass** sie einen pH-Wert im Bereich von 4.0 bis 5.0 aufweist.

12. Verwendung des Mittels nach einem der vorhergehenden Ansprüche zur langanhaltenden Konditionierung von menschlichem Haar, insbesondere zur Verbesserung der Kämmbarkeit und Geschmeidigkeit von menschlichem Haar.

13. Verfahren zur langanhaltenden Konditionierung von menschlichem Haar, **dadurch gekennzeichnet, dass** eine wässrige Zusammensetzung nach einem der Ansprüche 1 bis 11 auf das Haar aufgetragen wird, gegebenenfalls für einen Zeitraum von 1 bis 15 Minuten auf dem Haar belassen wird, gegebenenfalls vom Haar abgespült wird und die Haartemperatur auf den Bereich von 40 bis 140°C erhöht wird.

14. Verfahren nach Anspruch 13, **dadurch gekennzeichnet, dass** das Haar mit einem Haartrockner, mit einem digitalen Haarheizgerät, mit einem Glätteisen, das entweder flach oder mit bestimmten Oberflächenstrukturen versehen sein kann, oder mit einem runden Glätteisen erwärmt wird.

15. Kit für Haare, **dadurch gekennzeichnet, dass** es eine wässrige Zusammensetzung nach einem der Ansprüche 1 bis 11 und eine Vorrichtung enthält, die die Erhöhung der Haartemperatur ermöglicht.

## Revendications

1. Composition aqueuse pour les cheveux humains **caractérisée par le fait qu'**elle comprend un ou plusieurs polymères d'ammonium quaternaire cationiques ayant une densité de charge cationique de 3.0 mEq/g ou plus et un ou plusieurs tensioactifs non ioniques choisis parmi les suivants
a- éthoxylates d'alcools gras de la structure générale suivante
R₁ (OCH₂CH₂)ₙ OH
où R₁ est une chaîne alkyle linéaire ou ramifiée, saturée ou insaturée, qui peut être synthétique ou naturelle, avec une longueur de chaîne en C comprise entre 8 et 40,
b- éther de polypropylène glycol d'alcools gras selon la structure générale
R₂ (OCH₂ (CH₃)CH₂)ₙ OH
où R₂ est un alcool gras linéaire ou ramifié, saturé ou insaturé, qui peut être synthétique ou naturel, avec une longueur de chaîne en C comprise entre 8 et 40,
c- esters d'acides gras du polyéthylène glycol de la structure générale suivante
R₃ C(O) (OCH₂CH₂)ₙ OH
où R₃ est un groupe alkyle linéaire ou ramifié, saturé ou insaturé, qui peut être synthétique ou naturel, avec une longueur de chaîne en C comprise entre 7 et 39,
d- esters d'acides gras du polypropylène glycol ayant la structure générale suivante
R₄ C(O) (OCH₂(CH₃) CH₂)ₙ OH
où R₄ est un groupe alkyle linéaire ou ramifié, saturé ou insaturé, qui peut être synthétique ou naturel, avec une longueur de chaîne en C comprise entre 7 et 39,
e- éther de polyéthylène glycol et de polypropylène glycol d'alcools gras ayant la structure générale suivante
R₅ (OCH₂ (CH₃)CH₂)ₙ₁ (OCH₂CH₂)ₙ₂ OH
dans lequel R₅ est un groupe alkyle linéaire ou ramifié, saturé ou insaturé, qui peut être synthétique ou naturel avec une longueur de chaîne en C comprise entre 7 et 39, et n₁ et n₂ peuvent être identiques ou différents et représentent un nombre compris entre 1 et 40 à une concentration totale comprise entre 0.2 et 4% en poids, calculée par rapport à la composition totale, et
une ou plusieurs silicones aminées selon la structure générale 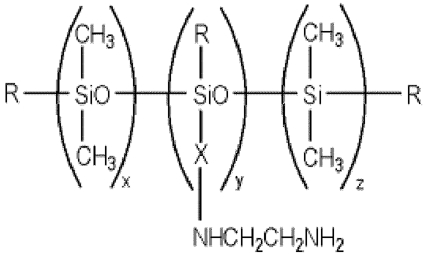
dans lequel R est identique ou différent de OH ou CH₃ ou OCH₃ et X représente le butyle, le propyle, l'isopropyle ou l'isobutyle, et
son pH est compris entre 3.0 et 5.0.

2. La composition selon la revendication 1 **caractérisée par le fait que** le polymère d'ammonium quaternaire cationique a une densité de charge de 3.5 à 8 mEq/g,.

3. Composition selon les revendications 1 et 2 **caractérisée par le fait que** le polymère d'ammonium quaternaire cationique est choisi parmi le Polyquaternium-37, le Polyquaternium-6, le Polyquaternium-7 et le Polyquaternium-16, de préférence le Polyquaternium-37.

4. Composition selon l'une quelconque des revendications 1 à 3 **caractérisée en ce qu'**elle comprend un ou plusieurs polymères cationiques d'ammonium quaternaire à une concentration totale de 0.1 à 5% en poids calculé par rapport à la composition totale.

5. La composition selon l'une quelconque des revendications 1 à 4 **caractérisée par le fait qu'**un ou plusieurs tensioactifs non ioniques sont choisis parmi
éthoxylates d'alcools gras de la structure générale suivante
R₁ (OCH₂CH₂)ₙ OH
où R₁ est une chaîne alkyle linéaire ou ramifiée, saturée ou insaturée, qui peut être synthétique ou naturelle, avec une longueur de chaîne en C comprise entre 8 et 40.

6. La composition selon l'une quelconque des revendications 1 à 5 **caractérisée en ce qu'**elle comprend un ou plusieurs tensioactifs non ioniques à une concentration totale de 0.25 à 3% en poids, calculée par rapport à la composition totale.

7. La composition selon l'une des revendications 1 à 6 **caractérisée par le fait qu'**elle comprend une ou plusieurs silicones aminées selon la structure générale 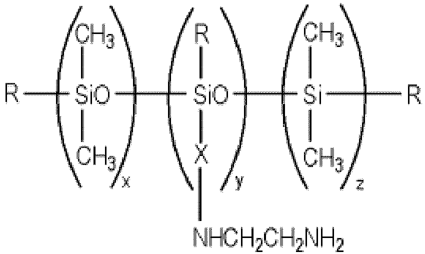 où R est le même ou différent OH ou CH₃ ou OCH₃ et X représente le butyle, le propyle, l'isopropyle ou l'isobutyle à une concentration de l'ordre de 0.1 à 5% en poids calculé par rapport à la composition totale.

8. Composition selon l'une des revendications 1 à 7 **caractérisée par le fait que** le silicone aminé est le Bis(Hydroxy/Methoxy) Amodimethicone.

9. Composition selon l'une des revendications 1 à 8 **caractérisée en ce qu'**elle ne comprend pas de tensioactif à base d'ammonium quaternaire à une concentration supérieure à 10%, en poids de la concentration totale d'un ou plusieurs polymères cationiques à base d'ammonium quaternaire ayant la densité de charge cationique définie dans les revendications précédentes.

10. La composition selon l'une quelconque des revendications précédentes **caractérisée par le fait qu'**elle comprend un ou plusieurs des composés suivants
- Solvant organique,
- Alcool gras de structure générale
R -OH₆
où R₆ est une chaîne alkyle linéaire ou ramifiée, saturée ou insaturée, qui peut être synthétique ou naturelle, avec une longueur de chaîne C comprise entre 12 et 24, qui peut également être substituée par un ou plusieurs groupes sur la chaîne alkyle,
- Filtre UV,
- Colorant direct pour cheveux choisi parmi les colorants cationiques et neutres,
- Extrait naturel de plantes,
- Hydrolysat de protéines,
- Ubiquinone, et
- Polymère non ionique.
- Huile et/ou substances huileuses choisies parmi les huiles de silicone, les huiles naturelles et synthétiques, les éthers dialkyles d'alcools gras et les esters d'alcools gras d'acides gras.

11. La composition selon l'une quelconque des revendications précédentes **caractérisée par** un pH compris entre 4.0 et 5.0.

12. Utilisation de la composition selon l'une quelconque des revendications précédentes pour le conditionnement durable des cheveux humains, notamment pour améliorer la coiffabilité et la souplesse des cheveux humains.

13. Procédé de conditionnement durable des cheveux humains **caractérisé en ce qu'**une composition aqueuse selon les revendications 1 à 11 est appliquée sur les cheveux, éventuellement laissée sur les cheveux pendant une période de 1 à 15 min, éventuellement rincée des cheveux et la température des cheveux est augmentée dans la gamme de 40 à 140°C.

14. Le procédé selon la revendication 13 **caractérisé par le fait que** les cheveux sont chauffés à l'aide d'un sèche-cheveux, d'un appareil numérique de chauffage des cheveux, d'un fer à repasser qui peut être plat ou avoir certaines structures de surface ou d'un fer à repasser de forme ronde.

15. Kit pour cheveux **caractérisé en ce qu'**il comprend une composition aqueuse selon les revendications 1 à 11 et un dispositif qui permet d'augmenter la température des cheveux.
